# EUROPEAN PATENT APPLICATION

(11) **EP 4 516 418 A1**
(43) Date of publication of application: **05.03.2025**
(21) Application number: 23195139.3
(22) Date of filing: 04.09.2023
(51) Int. Cl.: B09B 3/45, A61L 2/07, A61L 11/00, B09B 101/30, B09B 101/55, B09B 101/90

(54) **WASTE CARRYING CONTAINER AND EQUIPMENT FOR WASTE VOLUME EXPANSION STABILIZATION PROCESS**

(71) Applicant: Taiwan Steel Resources Co., Ltd., Shengang Township, Changhua County (TW)
(72) Inventor: FANG, Kenneth, Shengang Township (TW); CHEN, Wei-Chih, Shengang Township (TW)
(74) Representative: Contadin, Giorgio

(57) **Abstract**

Equipment adapted for a waste volume expansion stabilization process has an autoclave (10) and a waste carrying container (20). The waste carrying container (20) is configured for carrying a pile of waste (50), is positioned in the autoclave (10), and has a chamber (22) and a steam conducting unit (30) installed in the chamber (22) and having multiple steam tubes (31). Each steam tube (31) connects with the autoclave (10) via a first opening thereof and connects with the chamber (22) via a second opening thereof. High pressure steam flows into the steam tubes (31) from the first openings and flows into the chamber (22) to be mixed with the pile of the waste (50) via the second opening. The performance of the waste volume expansion stabilization process is improved.

## Description

### 1. Field of the Invention

The present invention relates to equipment for a recycling process of waste, and more particularly to a waste carrying container and related equipment adapted for a waste volume expansion stabilization process.

### 2. Description of Related Art

Before being recycled and reused, industrial waste, such as but not limit to slag, fly ashes, leaching residues, refractory lining materials, mining ores, tailing materials, etc., must be processed for stabilization to reduce unstable volume expansion components therein, to become reclamation products. Stabilized industrial waste can then be allowed to be added into concrete for reuse.

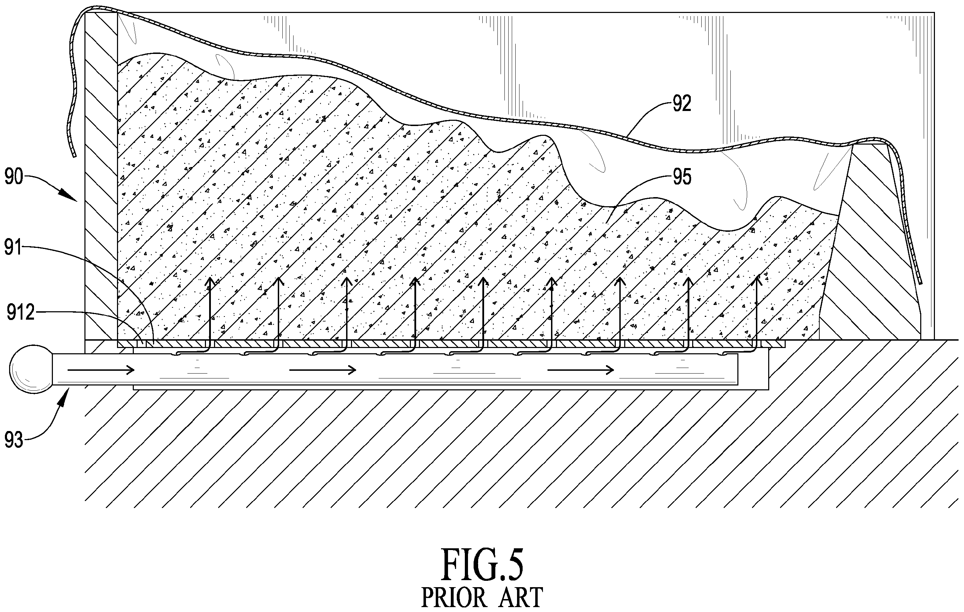

With reference to Fig. 5, in a conventional stabilization process of slag, a pile of slag 95 is dumped into a frame box 90. A support board 91 with multiple holes 912 is installed at a bottom of the frame box 90. Steam equipment 93 is installed below the support board 91. The frame box 90 is covered by a hood 92. The steam produced by the steam equipment 93 flows into the frame box 90 via the holes 912 of the support board 91 to proceed a hydration reaction in the slag 95 inside the frame box 90. Unstable components, such as calcium oxide, magnesium oxide, and other expansive components, contained in the slag 95 are eliminated and converted into stabilized components, such as calcium hydroxide, magnesium hydroxide, and other stabilized components.

However, the steam diffuses upwardly from the bottom of the support board 91 and is likely to be blocked by the pile of the slag 95 causing steam cannot diffuse freely to the upper layer in the frame box 90 in the existing current conventional stabilization process. Whereby, the slag 95 at the upper layer cannot be sufficiently mixed with the steam. The performance of stabilization of the slag 95 is poor and needs to be improved.

To overcome the shortcomings, the present invention's main purpose is to provide a waste carrying container and related equipment adapted for a waste volume expansion stabilization process. This invention can allow high pressure steam to be mixed homogenously with waste inside the carrying container to undergo intended waste stabilization process.

The present invention relates to a waste carrying container and related equipment adapted for a waste volume expansion stabilization process to allow a pile of waste carried by a waste carrying container to be evenly and sufficiently mixed with high pressure steam for the waste volume expansion stabilization process.

The waste carrying container is adapted for the waste volume expansion stabilization process, is configured for carrying a pile of waste, and comprises a chamber and a steam conducting unit. The steam conducting unit is installed in the chamber, connects with the chamber and exterior space out of the chamber, and comprises of multiple steam tubes extending into the chamber and arranged at spaced intervals. Each of the steam tubes has a first end, a second end, and each end has an opening. Each of the steam tubes connects with the exterior space of the chamber via the opening in the first end of the steam tube and connects with the internal space of the chamber via the opening in the second end of the steam tube.

Equipment adapted for a waste volume expansion stabilization process comprises an autoclave and a waste carrying container placed inside the autoclave. The waste carrying container comprises a chamber and a steam conducting unit. The steam conducting unit is installed in the chamber, connects with the autoclave and the chamber, and comprises multiple steam tubes extending into the chamber and arranged at spaced intervals. Each of the steam tubes has a first end, a second end, a first opening formed at the first end, and a second opening formed at the second end. Each of the steam tubes connects with a space of the autoclave via the first opening of the steam tube and connects with waste inside the chamber via the second opening of the steam tube.

Other objects, advantages, and novel features of the invention will become more apparent from the following detailed description when taken in conjunction with the accompanying drawings.

### IN THE DRAWINGS:

Fig. 1 is a side schematic view of equipment adapted for a waste volume expansion stabilization process in accordance with the present invention;
Fig. 2 is an enlarged example operational side schematic view of a first embodiment of a waste carrying container in Fig. 1;
Fig. 3 is an enlarged example operational side schematic view of a second embodiment of the waste carrying container in Fig. 1;
Fig. 4 is an enlarged example operational side schematic view of a third embodiment of the waste carrying container in Fig. 1; and
Fig. 5 is an operational schematic view showing an existing current conventional stabilization process of slag.

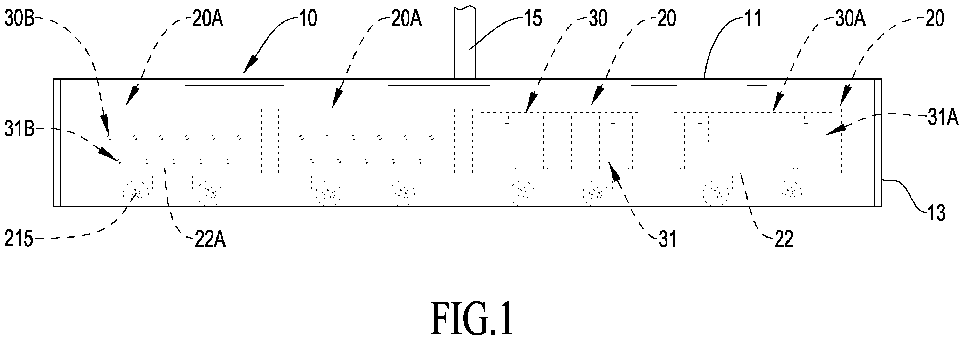

With reference to Figs. 1 to 4, equipment adapted for a waste volume expansion stabilization process in accordance with the present invention comprises an autoclave 10 and a waste carrying container 20, 20A. The waste carrying container 20, 20A is positioned in the autoclave 10 and is configured for carrying a pile of waste 50, such as but not limit to slag, fly ashes, leaching residues, refractory lining materials, mining ores, tailing materials, and so on.

With reference to Fig. 1, the autoclave 10 comprises a body 11 and at least one cover 13. The body 11 has a horizontally positioned extending cylinder and is connected to external steam equipment via a pipe 15. High pressure steam produced by the external steam equipment flows into the body 11 via the pipe 15. The body 11 may have an opening formed at one end thereof or two openings formed at two ends thereof. The amount of the cover 13 is the same as the amount of the openings of the body 11. Each of the at least one cover 13 is able to open and close a respective one of the openings of the body 11.

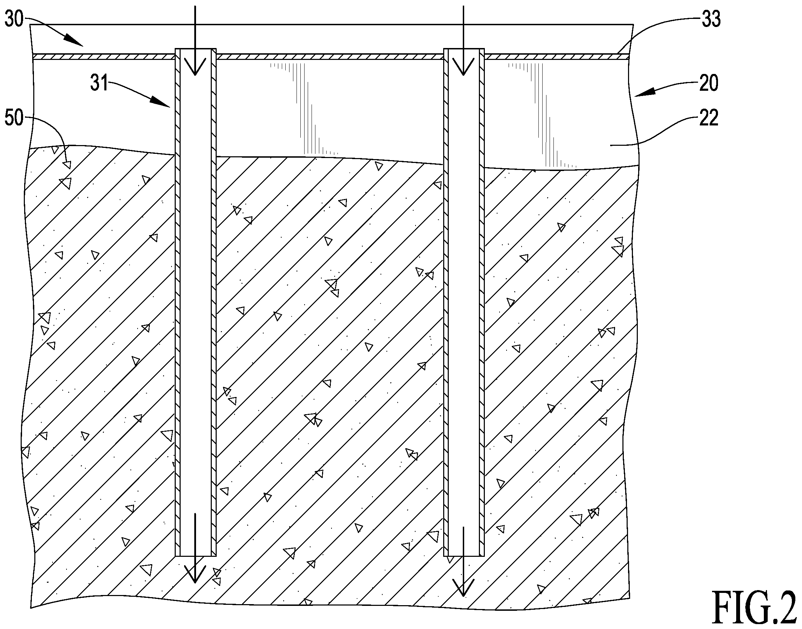

With reference to Figs. 1 and 2, a first embodiment example of the waste carrying container 20 comprises a chamber 22 and a steam conducting unit 30. The chamber 22 has an opening formed at a top thereof to allow the pile of the waste 50 to be filled into the chamber 22 from the top of the chamber 22. The waste carrying container 20 may selectively comprise multiple wheels 215 mounted to the bottom of the waste carrying container 20 to allow a user to conveniently move the waste carrying container 20.

The steam conducting unit 30 is positioned in the chamber 22 of the waste carrying container 20 and connects with the chamber 22 and exterior space, that is, the steam conducting unit 30 connects with the autoclave 10 and the chamber 22. The steam conducting unit 30 comprises multiple vertical steam tubes 31 extending into the chamber 22 and arranged at spaced intervals. Each steam tube 31 may be made of metal and has a first opening formed in a first end thereof and a second opening formed in a second end thereof. The first end is located higher than the second end. Each steam tube 31 connects with the exterior space of the chamber 22 via the first opening of the steam tube 31 and connects with the internal space of the chamber 22 via the second opening of the steam tube 31. The second end of each steam tube 31 is spaced apart from the internal surface of the chamber 22 to prevent the second opening of the steam tube 31 from being blocked by the internal surface of the chamber 22.

Preferably, each steam tube 31 has multiple evenly spaced holes formed through a peripheral surface of the steam tube 31 to increase the amount of opening holes of the steam tube 31 for the high pressure steam flowing out of the steam tube 31 into the chamber 22. Whereby, contacting surface of the waste 50 with the high pressure steam are increased, and the high pressure steam can be evenly and sufficiently mixed with the pile of the waste 50.

Multiple supporting racks may be positioned in the chamber 22 of the waste carrying container 20, be adjacent to the top of the chamber 22, and be arranged at spaced intervals. The steam conducting unit 30 comprises at least one tube holder 33 held on the multiple supporting racks. The multiple steam tubes 31 are fixed to the at least one tube holder 33 at spaced intervals and vertically extend into the chamber 22. The at least one tube holder 33 has multiple through holes spaced apart from one another for inserting the multiple steam tubes 31, respectively. The multiple steam tubes 31 may be fixed to the tube holder 33 via welding.

Lengths of the multiple steam tubes 31 are arranged according to a shape of the chamber 22; specifically, the second ends of the multiple steam tubes 31 are spaced apart from a bottom or a side of the internal surface of the chamber 22 to keep the second openings of the multiple steam tubes 31 from being blocked by the bottom or the side of the internal surface of the chamber 22.

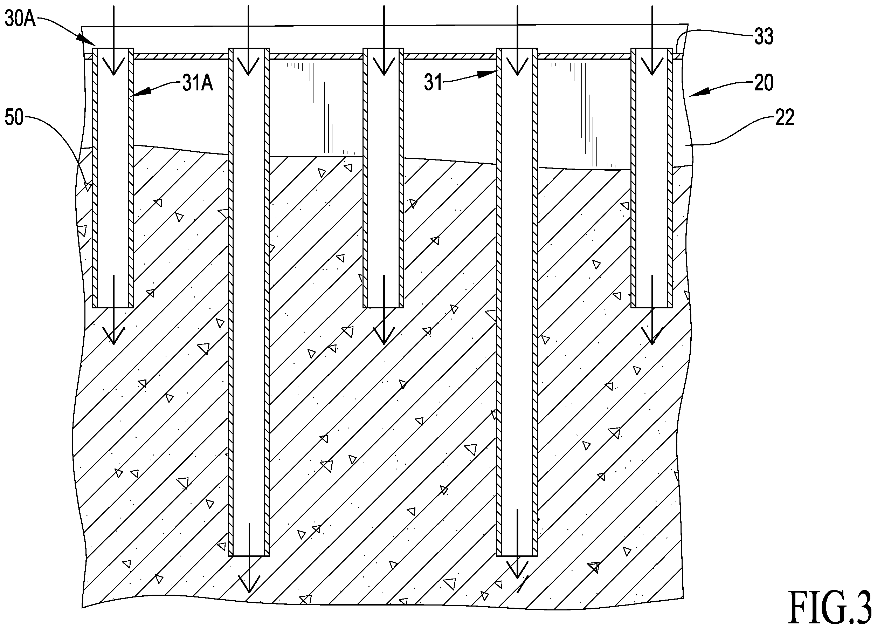

With reference to Figs. 1 and 3, in a second embodiment example of the waste carrying container 20, a steam conducting unit 30A comprises multiple evenly positioned steam tubes 31, 31A vertically extending into the chamber 22. A length of one of the multiple steam tubes 31A is shorter than another one of the multiple steam tubes 31. So, the second ends of the two steam tubes 31, 31A extending into the pile of the waste 50 carried by the chamber 22 are extended at different depths. Accordingly, the high pressure steam from the steam tubes 31, 31A with different lengths can flow into the pile of the waste 50 at different height to be evenly mixed with the pile of the waste 50.

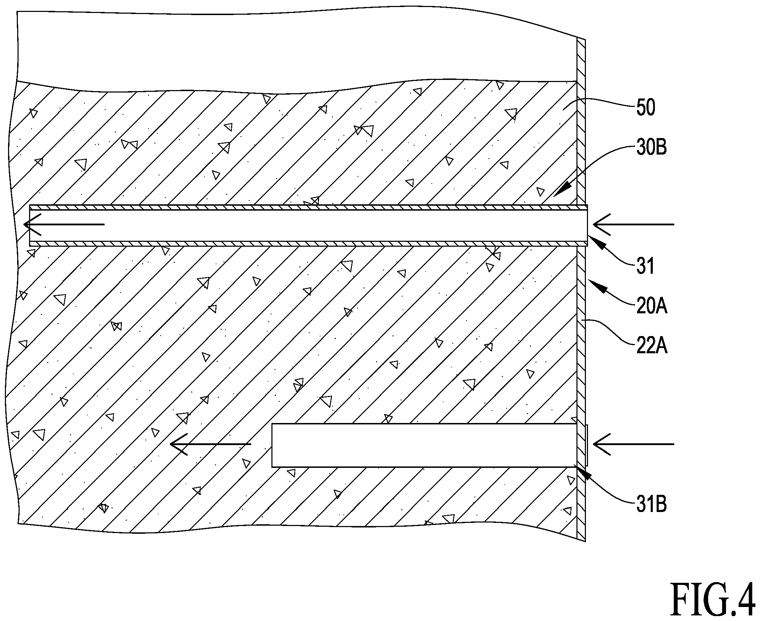

With reference to Figs. 1 and 4, a third embodiment example of the waste carrying container 20A comprise a chamber 22A and a steam conducting unit 30B. The steam conducting unit 30B comprises multiple steam tubes 31B horizontally extending into the chamber 22A and arranged at spaced intervals. The first end of each steam tube 31B is penetrated through a side wall of the chamber 22A. The first opening of each steam tube 31B formed in the first end connects with exterior space of the chamber 22A, that is, the first opening connects with the autoclave 10. The second end of each steam tube 31B is spaced apart from the internal surface of the chamber 22A to allow the steam tube 31B to connect with the internal space of the chamber 22A via the second opening formed at the second end of the steam tube 31B.

With reference to Figs. 1 to 4, after the pile of the waste 50 is filled into the chamber 22, 22A, the waste carrying container 20, 20A is placed into the autoclave 10, and the high pressure steam is injected into the autoclave 10. The high pressure steam in the autoclave 10 flows into the steam tubes 31, 31A, 31B via the first openings of the steam tubes 31, 31A, 31B, and then flows into the chamber 22, 22A via the second openings of the steam tubes 31, 31A, 31B to be evenly and sufficiently mixed with the pile of the waste 50 carried by the chamber 22, 22A for the waste volume expansion stabilization process.

When in use, as shown in Fig. 1, multiple waste carrying containers 20, 20A may be placed in the autoclave 10, so multiple piles of the waste 50 carried by the multiple waste carrying containers 20, 20A can be processed for the volume expansion stabilization at the same time. With the structures of the waste carrying containers 20, 20A, the high pressure steam inside the autoclave 10 can flow through the steam tubes 31, 31A, 31B from the first openings of the steam tubes 31, 31A, 31B and then flow into the chamber 22, 22A via the second openings of the steam tubes 31, 31A, 31B to be sufficiently mixed with the pile of the waste 50. Whereby, the steam can diffuse inside of the pile of the waste 50 to increase surface contact areas and to allow the steam to be evenly mixed with the pile of the waste 50. The performance of the waste volume expansion stabilization process is improved and a processing time for the waste volume expansion stabilization process can be reduced.

## Claims

1. A waste carrying container (20, 20A), adapted for a waste volume expansion stabilization process, configured for carrying a pile of waste (50), and **characterized in that** the waste carrying container (20, 20A) comprises:
a chamber (22, 22A); and
a steam conducting unit (30, 30A, 30B) installed and positioned inside the chamber (22, 22A), connecting with the chamber (22, 22A) and exterior space of the chamber (22, 22A), and comprising
multiple steam tubes (31, 31A, 31B) extending into the chamber (22, 22A) and arranged at spaced intervals, each of the steam tubes (31, 31A, 31B) having
a first end;
a second end;
a first opening formed at the first end; and
a second opening formed at the second end; wherein
each of the steam tubes (31, 31A, 31B) connects with the exterior space out of the chamber (22, 22A) via the first opening of the steam tube (31, 31A, 31B) and connects with the chamber (22, 22A) via the second opening of the steam tube (31, 31A, 31B).

2. The waste carrying container (20) as claimed in claim 1, wherein
each of the steam tubes (31, 31A) extends downwardly into the chamber (22); and
the first end of each of the steam tubes (31, 31A) is located higher than the second end of the steam tube (31, 31A).

3. The waste carrying container (20A) as claimed in claim 1, wherein
each of the steam tubes (31B) extends horizontally into the chamber (22A);
the first end of each of the steam tubes (31B) is penetrated through a side wall of the chamber (22A) to allow the first opening of the steam tube (31B) to connect with the exterior space out of the chamber (22A).

4. The waste carrying container (20, 20A) as claimed in any one of claims 1 to 3, wherein the second end of each of the steam tubes (31, 31A, 31B) is spaced apart from an internal surface of the chamber (22, 22A).

5. The waste carrying container (20, 20A) as claimed in any one of claims 1 to 3, wherein each of the steam tubes (31, 31A, 31B) has multiple holes formed through a peripheral surface of the steam tube (31, 31A, 31B) and connecting with the steam tube (31, 31A, 31B) and the chamber (22, 22A).

6. The waste carrying container (20, 20A) as claimed in any one of claims 1 to 3, wherein the waste carrying container (20, 20A) comprises multiple wheels (215) installed at a bottom of the waste carrying container (20, 20A).

7. Equipment adapted for a waste volume expansion stabilization process, **characterized in that** the equipment comprises:
an autoclave (10); and
a waste carrying container (20, 20A) positioned in the autoclave (10), and comprising
a chamber (22, 22A); and
a steam conducting unit (30, 30A, 30B) installed in the chamber (22, 22A), connecting with the autoclave (10) and the chamber (22, 22A), and comprising
multiple steam tubes (31, 31A, 31B) extending into the chamber (22, 22A) and arranged at spaced intervals, each of the steam tubes (31, 31A, 31B) having
a first end;
a second end;
a first opening formed at the first end; and
a second opening formed at the second end; wherein
each of the steam tubes (31, 31A, 31B) connects with the autoclave (10) via the first opening of the steam tube (31, 31A, 31B) and connects with the chamber (22, 22A) via the second opening of the steam tube (31, 31A, 31B).

8. The equipment adapted for the waste volume expansion stabilization process as claimed in claim 7, wherein
each of the steam tubes (31, 31A) extends downwardly into the chamber (22); and
the first end of each of the steam tubes (31, 31A) is located higher than the second end of the steam tube (31, 31A).

9. The equipment adapted for the waste volume expansion stabilization process as claimed in claim 8, wherein
each of the steam tubes extends (31B) horizontally into the chamber (22A);
the first end of each of the steam tubes (31B) is penetrated through a side wall of the chamber (22A) to allow the first opening of the steam tube (31B) to connect with the autoclave (10).

10. The equipment adapted for the waste volume expansion stabilization process as claimed in any one of claims 7 to 9, wherein the waste carrying container (20, 20A) comprises multiple wheels (215) installed at a bottom of the waste carrying container (20, 20A).

## Amended claims

### Amended claims in accordance with Rule 137(2) EPC.

1. A waste carrying container (20, 20A), adapted for an industrial waste volume expansion stabilization process, configured for carrying a pile of industrial waste (50), and **characterized in that** the waste carrying container (20, 20A) comprises:
a chamber (22, 22A) having an opening formed at a top of the chamber (22, 22A); and
a steam conducting unit (30, 30A, 30B) installed and positioned inside the chamber (22, 22A), connecting with the chamber (22, 22A) and exterior space of the chamber (22, 22A), and comprising
multiple steam tubes (31, 31A, 31B) extending into the chamber (22, 22A) and arranged at spaced intervals, each of the steam tubes (31, 31A, 31B) having
a first end;
a second end;
a first opening formed at the first end; and
a second opening formed at the second end; wherein
each of the steam tubes (31, 31A, 31B) connects with the exterior space out of the chamber (22, 22A) via the first opening of the steam tube (31, 31A, 31B) and connects with the chamber (22, 22A) via the second opening of the steam tube (31, 31A, 31B).

2. The waste carrying container (20) as claimed in claim 1, wherein
each of the steam tubes (31, 31A) extends downwardly into the chamber (22); and
the first end of each of the steam tubes (31, 31A) is located higher than the second end of the steam tube (31, 31A).

3. The waste carrying container (20A) as claimed in claim 1, wherein
each of the steam tubes (31B) extends horizontally into the chamber (22A);
the first end of each of the steam tubes (31B) is penetrated through a side wall of the chamber (22A) to allow the first opening of the steam tube (31B) to connect with the exterior space out of the chamber (22A).

4. The waste carrying container (20, 20A) as claimed in any one of claims 1 to 3, wherein the second end of each of the steam tubes (31, 31A, 31B) is spaced apart from an internal surface of the chamber (22, 22A).

5. The waste carrying container (20, 20A) as claimed in any one of claims 1 to 3, wherein each of the steam tubes (31, 31A, 31B) has multiple holes formed through a peripheral surface of the steam tube (31, 31A, 31B) and connecting with the steam tube (31, 31A, 31B) and the chamber (22, 22A).

6. The waste carrying container (20, 20A) as claimed in any one of claims 1 to 3, wherein the waste carrying container (20, 20A) comprises multiple wheels (215) installed at a bottom of the waste carrying container (20, 20A).

7. Equipment adapted for an industrial waste volume expansion stabilization process, **characterized in that** the equipment comprises:
an autoclave (10); and
a waste carrying container (20, 20A) positioned in the autoclave (10), and comprising
a chamber (22, 22A) having an opening formed at a top of the chamber (22, 22A); and
a steam conducting unit (30, 30A, 30B) installed in the chamber (22, 22A), connecting with the autoclave (10) and the chamber (22, 22A), and comprising
multiple steam tubes (31, 31A, 31B) extending into the chamber (22, 22A) and arranged at spaced intervals, each of the steam tubes (31, 31A, 31B) having
a first end;
a second end;
a first opening formed at the first end; and
a second opening formed at the second end; wherein
each of the steam tubes (31, 31A, 31B) connects with the autoclave (10) via the first opening of the steam tube (31, 31A, 31B) and connects with the chamber (22, 22A) via the second opening of the steam tube (31, 31A, 31B).

8. The equipment adapted for the industrial waste volume expansion stabilization process as claimed in claim 7, wherein
each of the steam tubes (31, 31A) extends downwardly into the chamber (22); and
the first end of each of the steam tubes (31, 31A) is located higher than the second end of the steam tube (31, 31A).

9. The equipment adapted for the industrial waste volume expansion stabilization process as claimed in claim 8, wherein
each of the steam tubes extends (31B) horizontally into the chamber (22A);
the first end of each of the steam tubes (31B) is penetrated through a side wall of the chamber (22A) to allow the first opening of the steam tube (31B) to connect with the autoclave (10).

10. The equipment adapted for the industrial waste volume expansion stabilization process as claimed in any one of claims 7 to 9, wherein the waste carrying container (20, 20A) comprises multiple wheels (215) installed at a bottom of the waste carrying container (20, 20A).
